# EUROPEAN PATENT APPLICATION

(11) **EP 2 305 339 A1**
(43) Date of publication of application: **06.04.2011**
(21) Application number: 10181282.4
(22) Date of filing: 28.09.2010
(51) Int. Cl.: A61M 25/06, A61B 19/00, A61M 39/02

(54) **Telescopic guide device for catheters to facilitate their insertion into the human cardiovascular system**

(30) Priority: 30.09.2009 IT BO20090073 U
(71) Applicant: Tre Esse Progettazione Biomedica S.r.l, 40138 Bologna (IT)
(72) Inventor: Plicchi, Gianni, I-40125, BOLOGNA (IT); Marcelli, Emanuela, I-40125, BOLOGNA (IT); Cercenelli, Laura, I-40138, BOLOGNA (IT); Panfili, Mauro, I-40139, BOLOGNA (IT); Golinelli, Daniele, I-40131, BOLOGNA (IT)
(74) Representative: Porsia, Dino

(57) **Abstract**

Telescopic guide device (7) with an adapter (10), for mounting on catheters (E) to facilitate their insertion and movement in the human cardiovascular system by a manual procedure or by means of a robotic manipulator, **characterized in that** the telescopic guide assembly (7) is provided at its other end with an adapter means having a friction coupling (1, 101, 201, 201', 301, 2, 3, 4), to be mounted on the proximal portion of the catheter which is connected to the handle (M) of the catheter, in such a way that it can be associated with catheters made by different manufacturers and also with catheters having different diameters which lie within a wide range of values.

## Description

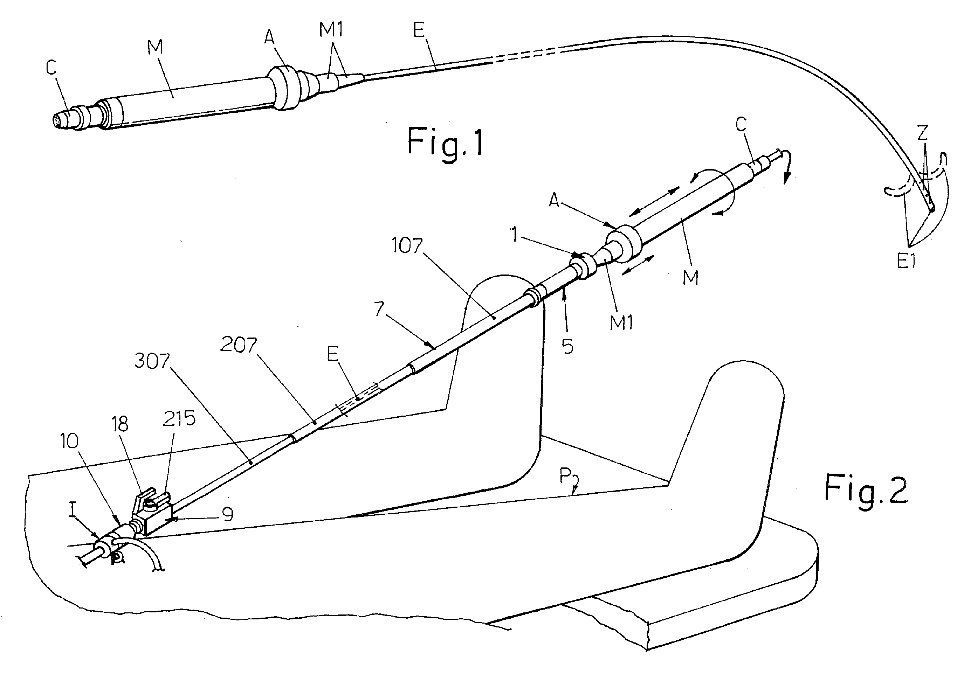

There is a known way of inserting flexible catheters into the cardiovascular system of the human body for diagnostic and/or therapeutic purposes, the catheters being, for example, of the type illustrated in Figure 1 of the appended drawings, each catheter being provided with a robust handle M whose front end M1 is fixed to the catheter E and whose rear end carries a connector C for connection to the diagnostic instruments. If required, an annular slider A can be made to slide in both directions on this handle, thus causing the deflection of the distal end E1 of the catheter by means of known connections inside the catheter, as indicated in broken lines in Figure 1, to produce the deflection manoeuvres required during the insertion of the catheter into the human cardiovascular system and its removal therefrom. In normal use, appropriate movements are imparted to the handle M to make the catheter advance or withdraw by axial movements or rotate about its axis in both directions, and furthermore, as mentioned above, if said slider A is present it can be operated to subject the catheter, if required, to an operation of deflection or steering of its distal end which is usually provided with one or more electrodes Z. To facilitate the maneuvering of a catheter according to the example of Figure 1, there is a known way of using an insertion and adaptation device as described in Italian patent 1363313 and in European publication No. 2 218 474 and corresponding US Application No. US-2010-0204680 A1, to which ample reference will be made. This device comprises a telescopic guide which is to be mounted on the catheter in advance, one end of this guide being designed for coupling to the end M1 of the catheter handle as shown in the example of Figure 1, while its other end is coupled to the introduction device whose cannula has previously been inserted into the patient's artery or vein, with the interposition of an adapter means which is fitted in a sealed way into the resilient seal of the introduction device and which carries its own sealing means for calibrated interaction with the diameter of the catheter, in such a way that the catheter can be made to slide and can rotate with a low degree of friction through the adaptation and introduction unit, while the portion of catheter lying between said unit and the handle M is kept rigid by said telescopic guide and is therefore in the optimal condition for reacting to the commands imparted to it through the handle M. The known device to which reference is made presents difficulties in the coupling of the telescopic guide to the handle M, since, for any given outside diameter of the catheter E, the shape and dimensions of the front end M1 of the handle M may vary from one manufacturer to another. Another limitation of the known device is that the coupling between the telescopic guide and the handle M of the catheter is such that the two parts form a single rigid body, and this condition gives rise to difficulties in the positioning and manoeuvring of the catheter.

In a preferred embodiment of the known device, the telescopic guide is formed by only two sections, and therefore the guide is excessively long when in its closed position, which complicates the packaging of the device in a sterile package and also complicates the use of the device.

Another limitation of the known device arises from the lack of reliability and security of the male and female screw means which is proposed for axially locking the catheter with respect to said adapter, since the activation and inactivation of this screw means takes a considerable time, and the screw means also acts directly in contact with the catheter, with the risk of damaging it.

The object of the invention is to overcome all of these and other problems and limitations of the prior art with an improved device as claimed in Claim 1 and in the subsequent dependent claims, the features and advantages of this device being made evident by the following description which refers to the figures of the three appended sheets of drawings, in which, in addition to Figure 1 which has already been described,
- Fig. 2 is a perspective view of the device in use, with the catheter being inserted into the cardiovascular system of a patient;
- Figs. 3 and 4 show the device from above and from the side, with parts shown in section and with the telescopic guide shown in the closed position in which it is taken from the sterile packaging;
- Figs. 5 and 6 show in side view, with parts in section, the part of the device with the adapter and the axial catheter locking means, in the rest position and in the working position respectively;
- Fig. 7 is a perspective view of the rotating stopping member of the locking means of Figures 5 and 6.

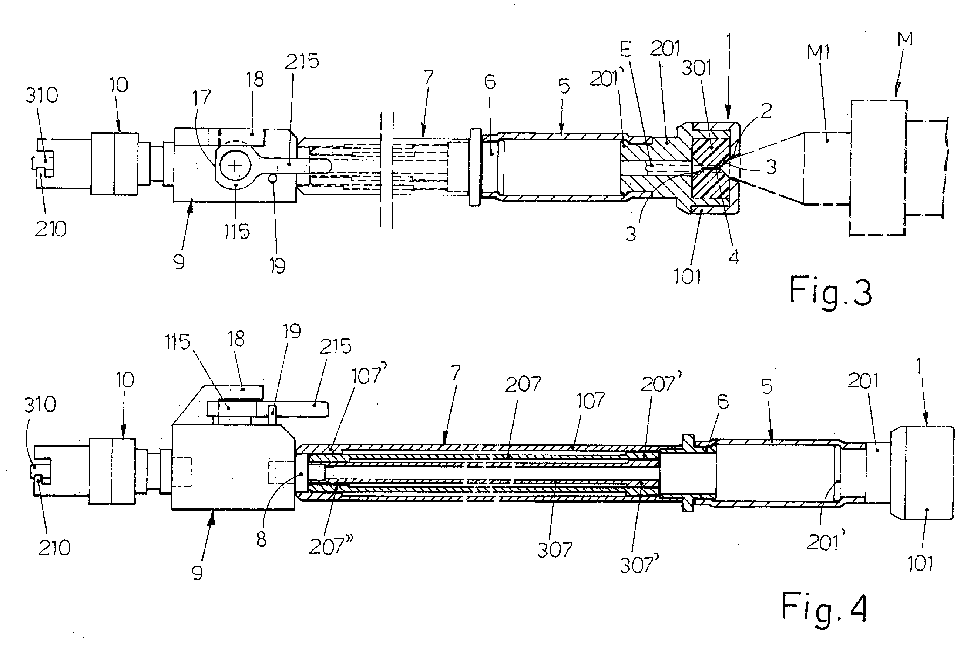

Figures 2, 3 and 4 show that the novel device comprises a first unit 1 for fixing the device directly to the first portion of the catheter which extends from the handle M, this unit comprising a small boxlike body 101 with a small hollow coupling member 201 at one end, with an axial aperture 2 at the other end, and containing a body 301 made from suitable elastomeric material, having central recessed indentations 3 on its opposite faces and having a diametrically located flat or shaped penetrating slit 4 in the portions where these indentations are present, through which the catheter E can be inserted with a friction fit until the unit 1 reaches the end M1 of the handle M, as shown by way of example in Figure 3, in such a way that the unit is in contact with a portion of the catheter which executes no bending movements and on which the unit 1 remains in a stable way and in axial alignment as a result of the friction fit. The diameter of the axial cavity of the coupling member 201 and the dimensions of the slit 4 are such that the unit 1 can be positioned on catheters whose outside diameter lies within a wide range of values and on catheters having handles M of any shape or size, thus yielding benefits in terms of practicality and economy.

The coupling member 201 terminates in an enlarged edge with a curved profile 201', in such a way that the end of a tube 5, made from silicone rubber for example and preferably transparent to reveal the passage and movement of the catheter, can be frictionally fitted in a sealed way on to the coupling member, while its opposite end is frictionally connected in a sealed way to a similar coupling member 6 fixed to one end of the telescopic guide 7 which is described more fully below, the whole arrangement being such that, before reaching this guide 7, the catheter slides in a guided way in the flexible tube 5 which acts as a joint between the unit 1 and the guide 7, in order to allow greater freedom in the positioning of the catheter handle M and to prevent the direct transmission of small movements, which are imparted to the handle but which are not necessary for the operation of the catheter, to the guide 7 and to the downstream devices or to the patient P, which may occur in the prior art. A further improvement made to the device is that the telescopic guide 7 is made with more than two telescopic sections, for example with three sections as in the example shown in Figure 4, in such a way that, when at rest with the three sections retracted into each other, the guide is shorter than a guide with two sections which have a similar length when extended, since the length in the closed condition is substantially equal to the length of the guide in the extended condition divided by the number of telescopic sections that make up the guide. Figure 4 shows that the aforesaid coupling member 6 is fixed to one end of the outer section 107 of the guide 7 and that the coupling extends slightly into this section so as to halt the contraction of the intermediate section 207 which has an outer enlarged area 207' which is halted in the course of extension when it encounters the inner enlarged area 107' of the outer section 107. The intermediate section is also provided at its other end with an inner enlarged area 207" which acts as a stop on the outer enlarged area 307' of the inner section 307 which has a coupling 8 fixed on its outer end and formed from opposing coupling members, through which the catheter can pass axially, while the other end of the coupling is fixed to the body of the catheter braking unit 9 whose opposite face is fixed to the known adapter 10 which was mentioned in the preamble. Advantageously, the outside diameter of the body of the coupling 8 is such that the distal end of the outer section 107 of the guide 7 can be frictionally fitted on to this body, in such a way that the guide remains stably in the closed position when the device is removed from the packaging, in order to facilitate the application of the device. In the telescopic guide 7 as shown in Figure 4, the inner sections 207 and 307 are mounted in the outer section 107 from the end of this section which is temporarily free of the coupling 6 which is applied and fixed subsequently. When the three sections have been coupled together, the coupling 8 is applied and fixed to the outer end of the section 307, and thus the guide is substantially formed by at least five parts. However, it is to be understood that the construction of the telescopic guide 7 can be varied and modified in numerous ways without departing from the protective scope of the invention.

Figures 3, 4 and 5 show that the braking unit 9 comprises a body 109 pierced longitudinally by a hole which has, for example, a portion of smaller diameter 11 which can be engaged by one of the end coupling members of the coupling 8, a tube 13 of suitable plastic material, such as silicone rubber, being initially inserted into the remaining portion 12 of said hole and being stopped by the step formed by the hole 11, after which the coupling member 110 of the adapter 10 is inserted and fixed. The inside diameter of the tube 13 is made suitably larger than the outside diameter of the catheter. The body 109 has a hole 14 running perpendicularly to the intermediate part of the portion of the hole 12 occupied by the tube 13, the hole 14 being rotatably engaged by a barrel 15 having a head 115 and a radial lever 215 which remain outside said body 109. On its lower end the barrel 15 has a recess 16, in the form of a sector of a circle for example, the generatrix of which is parallel to the longitudinal axis of the lever 215, such that, when this lever is aligned longitudinally with the guide 7, as shown in solid lines in Figures 3, 4 and 5, said recess 16 is positioned longitudinally above the tube 13, substantially without deformation of the latter, while the end appendages 116 of the recess 16 are placed at the sides of the tube 13 and are **characterized in that** they have suitably rounded edges.

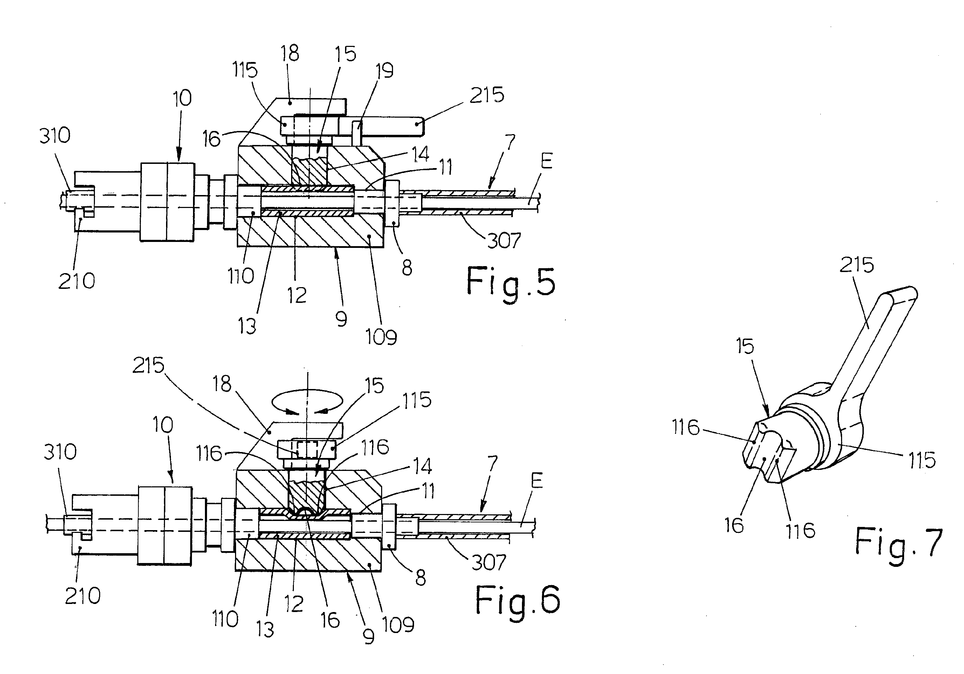

Figure 3 shows that the head 115 of the barrel 15 is provided, laterally and over its whole height, with a flat 17 which reduces its diameter and which, during the assembly of the unit 9, when the barrel is inserted into its seat 14, is positioned parallel to and on the same side as a cam 18 in the shape of an L rotated through 90°, which is fixed to the body 109, in such a way that, when the lever 215 is subsequently rotated into alignment with the holes 11 and 12, the head 115 of the barrel is positioned under the upper arm of said cam 18, thus keeping the barrel 15 stably in position. When the barrel 15 has been fitted, a stop pin 19 is positioned and fixed in a suitable seat in the body 109, said lever 215 bearing on this pin when it is in said longitudinal orientation in which it does not flatten the rubber tube 13, thus leaving the catheter free to slide through the unit 9 and through the upstream units 1 and 7 and the downstream unit 10. If the operator rotates the lever 215 through 90°, the lever is positioned under the arm of the cam 18, which contributes to the axial retention of the barrel 15 in position, since, as a result of this rotation, the lateral appendages 116 of the end recess 16 of the barrel press on the tube 13 flatten it as shown in Figure 6, and this flattening of the tube 13 gently locks the catheter E and prevents it from moving in an undesirable way in relation to all the components of the device in question. It is to be understood that the scope of the invention also covers solutions different from that which has been described for achieving the flattening of the tube 13, for example the use of rocking control means which, with the assistance of resilient means, enable the position of the barrel or stopping member 15 to be modified in its seat 14.

The adapter 10 is of the type fully described in the prior patents cited in the preamble and is provided with a bayonet fitting 210 for rapid connection to the introduction device I of Figure 2 and with an axial nozzle 310, from which the catheter E emerges by a sliding movement, and which is designed to be coupled to the seal of the introduction device I, which is not illustrated in detail since it is a known means.

All the components of the device are made from non-toxic materials, which are compatible with the use for which the device is intended and can be easily sterilized.

## Claims

1. Telescopic guide device (7) with an adapter (10), for mounting on catheters (E) to facilitate their insertion and movement in the human cardiovascular system by a manual procedure or by means of a robotic manipulator, **characterized in that** the telescopic guide assembly (7) is provided at its other end with an adapter means having a friction coupling (1, 101, 201, 201', 301, 2, 3, 4), to be mounted on the proximal portion of the catheter which is connected to the handle (M) of the catheter, in such a way that it can be associated with catheters made by different manufacturers and also with catheters having different diameters which lie within a wide range of values.

2. Device according to Claim 1, in which said adapter means with a friction coupling (1) is connected to the telescopic guide (7) by means of a short jointed guide formed for example by a flexible tube (5).

3. Device according to Claim 2, in which said flexible tube (5) is transparent, thus revealing the passage of the catheter through it.

4. Device according to Claim 1, in which the telescopic guide (7) is formed by more than two sections, for example three sections (107, 207, 307).

5. Device according to Claim 4, in which the telescopic guide (7) comprises said telescopic sections (107, 207, 307) and comprises a first coupling (6) to be fixed on the end of the outer section (107) which is to be connected to said jointed guide (5) and comprises a second coupling (8) to be fixed on the outer end of the inner section (307) and to be fixed indirectly to the adapter (10), the body of this second coupling (8) having a length such that it can be fitted with a sufficient degree of friction into the free end of the outer section (107) of the guide, so as to stabilize the telescopic guide (7) in the closed position.

6. Device according to claim 5, in which said second end coupling (8) of the telescopic guide (7) is fixed to the adapter (10) with the interposition of a small unit (9) for braking the catheter, provided with a flexible sheath (13) through which the catheter passes and provided with rapid actuating means (15) which can be moved from a rest position to a position in which they flatten said sheath on to the catheter inside it, in order to brake the catheter gently in the desired position.

7. Device according to Claim 6, in which said rapid actuating means are rotary means (15, 115, 215, 16, 116).
